# EUROPÄISCHE PATENTSCHRIFT

(11) **EP 0 999 229 B1**
(45) Veröffentlichungstag und Bekanntmachung des Hinweises auf die Patenterteilung: **28.06.2006**
(21) Anmeldenummer: 99121018.8
(22) Anmeldetag: 21.10.1999
(51) Int. Cl.: C08G 65/332, C12P 7/62

(54) **Verfahren zur Herstellung von Acrylsäuereestern und/oder Methacrylsäureestern von Polyoxyalkylenen und deren Verwendung**
Process for the preparation of acrylic acid esters and/or methacrylic esters of polyoxyalkylenes and their uses
Procédé pour la préparation des esters d'acide acrylique et/ou méthacrylique et polyoxyalkylènes et leur usage

(30) Priorität: 03.11.1998 DE 19850541
(43) Veröffentlichungstag der Anmeldung: 10.05.2000
(73) Patentinhaber: Goldschmidt GmbH, 45127 Essen (DE)
(72) Erfinder: Grüning, Burghard, Dr., 45134 Essen (DE); Hills, Geoffrey, 45355 Essen (DE); Josten, Wolfgang, Dr., 53639 Königswinter (DE); Schaefer, Dietmar, Dr., 45529 Hattingen (DE); Silber, Stefan, Dr., 47804 Krefeld (DE); Weitemeyer, Christian, Dr., 45134 Essen (DE)

(56) Entgegenhaltungen:
- WO-A-97/11982
- US-A- 5 736 606
- DATABASE WPI Section Ch, Week 199833 Derwent Publications Ltd., London, GB; Class A14, AN 1998-379985 XP002129901 & JP 10 150981 A (TOYO INK MFG CO LTD), 9. Juni 1998 (1998-06-09)
- DIE MAKROMOL. CHEMIIE- RAPID COMMUNICATIONS, Bd. 2, Nr. 6/7, August 1981 (1981-08), Seiten 427-434, XP002129899
- CHEMICAL ABSTRACTS, vol. 102, no. 21, Mai 1985 (1985-05) Columbus, Ohio, US; abstract no. 183757u, NITTO ELECTRIC CO. LTD.: Seite 473; XP002129900 & JP 59 220196 A (NITTO ELECTRIC CO. LTD.)

## Beschreibung

Gegenstand der vorliegenden Erfindung ist ein Verfahren zur Herstellung von Acrylsäureestern und/oder Methacrylsäureestern von Polyoxyalkylenen in Anwesenheit eines die Ver- oder Umesterung katalysierenden Enzyms sowie deren Verwendung.

Unter den Rohstoffen zur Herstellung von Polymerprodukten hat die Verarbeitung von Acrylmonomeren in den letzten Jahren eine stürmische Entwicklung genommen. Acrylmonomere finden überwiegend Einsatz bei der Herstellung von Fasern, Dispersionen, Lackrohstoffen, Klebrohstoffen und thermoplastischen Massen. Geringere Mengen dienen als Ausgangsstoffe für unterschiedliche chemische Synthesen.

In diesem Zusammenhang sind auch Polymere auf Basis von Acryl- und/oder Methacryloylpolyoxyalkylenen von zunehmendem Interesse. Durch gezielte Variation der Polyoxyalkyleneinheiten können Monomere mit einem maßgeschneiderten Löslichkeitsverhalten erhalten werden, die dann allein oder in Kombination mit anderen olefinisch ungesättigten Verbindungen zu Polymeren abreagieren können. Solche Verbindungen finden dann beispielsweise Verwendung als Hilfsstoffe zur Formulierung wäßriger Tinten, wie in der DE-A-196 54 752 beschrieben wird. Ein weiteres Einsatzgebiet solcher Verbindungen ist in der Dispergierung von Pigmenten zur Bereitung wasserverdünnbarer Beschichtungsstoffe zu suchen, wie dies in EP-A-0 803 556 und JP-A-092 670 34 beschrieben wird. Somit ist es nicht überraschend, wenn Acryl- und/oder Methacryloylpolyoxyalkylene auch kommerziell erhältlich sind (Fa. Nippon Oil and Fats Co.).

Im Sinne dieser Erfindung ist unter "Acryl" oder "Methacryl" ein Radikal der allgemeinen Formel 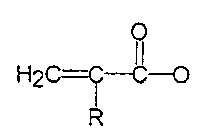 zu verstehen, wobei R = CH₃ oder H ist.

Verfahren zur Herstellung von Acryl- und/oder Methacryloylpolyoxyalkylenen sind bereits beschrieben.

Neben Verfahren zur Ver- und Umesterung von Acrylaten und/oder Methacrylaten, die im wesentlichen den literaturbekannten Herstellungsverfahren für Carbonsäureester entsprechen, wie sie beispielsweise in J. March, Advanced Organic Chemistry, Wiley, 1992 beschrieben sind, sind im Zusammenhang mit der Modifizierung von Polyoxyalkylenen auch speziell angepaßte Verfahren bekannt.

Man geht dabei oftmals von hydroxyfunktionellen Vorstufen aus und führt die Acryl- und/oder Methacryloylgruppe durch Veresterungs- oder Umesterungsverfahren, ausgehend von den entsprechenden Acryl- und/oder Methacrylsäureestern oder Acryl-und/oder Methacrylsäuren, ein. Im Regelfall werden dabei Metallsalze oder deren organische Komplexe oder Säuren verwendet. So beschreibt die DE-A-19 535 936 beispielsweise die Acrylierung von Polyetherpolyolen unter Katalyse von p-Toluolsulfonsäure und unterphosphoriger Säure unter Einsatz eines Schleppmittels und zusätzlicher Radikalfänger bei Temperaturen von 80 - 100 °C. Dieses und analoge Verfahren werden im Regelfall bei Temperaturen von oberhalb 80 °C, insbesondere oberhalb 100 °C, durchgeführt und bedürfen einer zusätzlichen Stabilisierung des Reaktionsgemisches durch Radikalfänger (zum Beispiel Methylhydrochinon), um eine unerwünschte Polymerisation der Acryloyl- und/oder Methacryloylverbindungen bei diesen Temperaturen verläßlich zu unterdrücken. Der Katalysator muß anschließend für viele Anwendungsgebiete entfernt oder aber zumindest neutralisiert werden, um unerwünschte Folgereaktionen zu vermeiden. Dies erfordert einen aufwendigen Aufarbeitungsprozeß, indem man Metalloxide, Metallhydroxide oder entsprechende Salze der Metalle beziehungsweise der als Katalysator verwendeten Säuren bildet und anschließend im Regelfall abfiltriert. Derartige Filtrationen acryl- und/oder methacryloylhaltiger Reaktionsgemische sind technologisch und arbeitssicherheitstechnisch aufwendig und häufig langwierig. Bedingt durch die hohe Temperatur sind so hergestellte acryl- und/oder methacryloylfunktionelle Verbindungen häufig intensiv gefärbt (gelb bis braun-schwarz). Dies ist für eine direkte Verwendung solcher Acryl- und/oder Methacryloylverbindungen in Anwendungen mit erhöhten Anforderungen mit Bezug auf die Färbung der eingesetzten Rohstoffe oft prohibitiv (zum Beispiel Verwendung als Reaktivverdünner in strahlenhärtenden Klarlacken oder Verwendung als Rohstoff für Polyacrylate für die Kosmetikindustrie).

Um einige der oben beschriebenen Nachteile zu umgehen, beschreibt US-A-4 528 334 wie in einer Eintopfreaktion die Polymerisation von Acrylsäure in Gegenwart von Polyoxyalkylenen durchgeführt werden kann, wobei man durch Temperaturen oberhalb 145 °C die Bildung entsprechender polyoxyalkylenmodifizierter Polyacrylsäure erreicht. Bei diesem Verfahren sind ebenfalls die benötigten hohen Temperaturen als nachteilig zu nennen. Außerdem würde beim Einsatz weiterer Monomerer aus der wichtigen und weitverbreiteten Familie der Acryl- und/oder Methacrylsäureester eine unkontrollierbare Folge von Umesterungsreaktionen stattfinden, die das Verfahren somit mit Bezug auf seine Variabilität sehr einschränken.

Um auch unter milden Reaktionsbedingungen zu zufriedenstellenden Ausbeuten zu kommen, bedient man sich häufig besonders reaktiver Acryl- und/oder Methacrylsäurederivate. Umsetzungen von Polyoxyalkylenverbindungen mit Acryl- und/oder Methacrylsäurehalogeniden, im Regelfall -chloride, werden u. a. in Polym. J., Vol. 17, 827 ff., Polym. Bull., Vol. 15, 425 ff. und Colloid Polym. Sci., Vol. 275, 227 - 233 beschrieben.

Die Reaktion von Acryl- und/oder Methacrylsäureanhydriden mit Polyoxyalkylenverbindungen wird in Macromolecules, Vol. 30, 6489 - 6493 beschrieben.

Diese Verfahren sind insbesondere durch die Handhabbarkeit der reaktiven Acryl- und/oder Methacrylsäurehalogenide und -anhydride in ihrem Einsatzspektrum begrenzt. Speziell die Anforderungen an die Lagerungsbedingungen, den notwendigen Ausschluß geringster Feuchtigkeitsspuren und auch an die allgemeinen Arbeitsschutzbedingungen sind so hoch, daß einer kommerziellen Nutzung solcher Verfahren ein oft nicht zu gerechtfertigender Aufwand gegenübersteht.

R. Tor, Enzyme Micro. Technol., 1990, Vol. 12, April, S. 299 - 304, beschreibt die enzymatisch katalysierte Umesterung von Acryl- und Methacrylmonomerestern zur Herstellung von Hydroxy- und Dihydroxyalkylacrylaten und -methacrylaten ohne Bildung von Di- oder Triacrylat und -methacrylaten. Insbesondere werden 2-Hydroxyethyl-, 2-Hydroxypropyl- und 1,2-Dihydroxypropylester von Acrylsäure und Methacrylsäure untersucht.

Die Aufgabe der vorliegenden Erfindung besteht in der Bereitstellung einer vereinfachten Veresterung oder Umesterung von Acryl- und/oder Methacrylsäure oder Acryl-und/oder Methacrylsäureestern mit Polyoxyalkylenen sowie in der Bereitstellung der so erhältlichen Reaktionsprodukte. Ein solches Herstellungsverfahren soll insbesondere eine deutlich hellere Farbe der Reaktionsprodukte ermöglichen, die Bildung von Nebenprodukten (aufgrund unselektiver Katalyse) vermeiden, eine unkomplizierte Entfernung des Enzymkatalysators vom Produkt ermöglichen und unerwünschte und unkontrollierte radikalische Polymerisationen der Acryl- und/oder Methacryloylverbindungen vermeiden. Das Verfahren soll weiterhin keinerlei aufwendige Aufarbeitungsschritte benötigen und außerdem sollen auch keine arbeitssicherheitstechnischen Nachteile entstehen, wie sie bei der Verwendung sehr reaktiver Acryl- und/oder Methacrylsäurederivate auftauchen.

Die vorstehend genannte Aufgabe wird gelöst durch Acryloyl-und/oder Methacryloylverbindungen von Polyoxyalkylenen, erhältlich durch ein Verfahren zur Ver- oder Umesterung von Acryl- und/oder Methacrylsäure oder Acryl- und/oder Methacrylsäureestern in Anwesenheit eines die Ver- oder Umesterung katalysierenden Enzyms mit Polyoxyalkylenen der allgemeinen Formel (I) 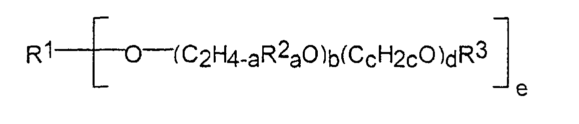 wobei
R¹ = ein Wasserstoff, ein e-bindiger, gegebenenfalls verzweigter, cyclischer, ungesättigter und/oder aromatischer Kohlenwasserstoffrest, ein gegebenenfalls substituierter Aromat, ein Kohlenhydrat oder Kohlenhydratderivat ist,
R² = gleiche oder verschiedene Alkylreste oder Alkylenreste mit 1 bis 24 Kohlenstoffatomen oder gegebenenfalls substituierte Phenylreste mit bis zu 24 Kohlenstoffatomen,
R³ = ein Wasserstoffrest oder ein einwertiger organischer Rest,
a = 0 bis 3,
b = 0 bis 100,
c = 2 bis 12,
d = 0 bis 100,
e = 1 bis 30,
die Summe (b + d) = 4 bis 200 ist,
wobei pro Molekül wenigstens eine OH-Gruppe vorhanden ist und die Reihenfolge der Polyoxyalkylensegmente (C₂H₄₋ₐR²ₐO)_{b} und (C_{c}H_{2c}O)_{d} beliebig ist.

Der Index a kann in einem Polymer verschiedene Werte annehmen. Damit soll ausgedrückt werden, daß geeignete Polyoxyalkylene sowohl zum Beispiel Homopolymere von Ethylenglykol, Copolymere von Ethylenglykol und 1,2-Propylenglykol, aber auch ein mehrfaches Copolymer aus mehr als zwei Monomeren, wie Ethylenglykol, 1,2-Propylenglykol und 1,2-Butylenglykol, sein können. Unabhängig davon kann auch der Index c in einem Polymer verschiedene Werte annehmen, so daß zum Beispiel mehrfache Copolymere zusätzlich mit 1,4-Butylenglykol aufgebaut sein können. Die Copolymere können statistisch oder blockweise aufgebaut sein.

Es ist dem Fachmann geläufig, daß die Verbindungen in Form eines Gemisches mit einer im wesentlichen durch statistische Gesetze geregelten Verteilung vorliegen. Die Werte für die Indices b und d stellen deshalb Mittelwerte dar. Besonders bevorzugt im Sinne der vorliegenden Erfindung beträgt die Summe (b + d) = 8 bis 120.

Beispiele von Polyoxyalkylenen, die erfindungsgemäß durch enzymatisch katalysierte Ver- oder Umesterung mit Acryl-und/oder Methacrylsäureestern oder Acryl- und/oder Methacrylsäure umgesetzt werden können, sind:

H₂C=CH―(OCH₂CH₂)₁₀(OCH₂CHCH₃)₂₀OH

H₂C=CH―CH₂―(OCH₂CH₂)₄(OCH₂CHCH₃)₁₆OH

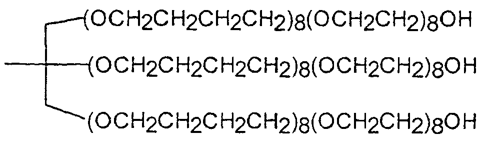

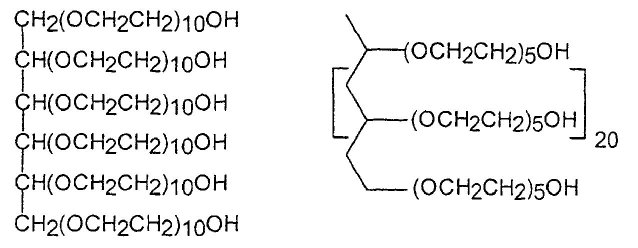

H―(OCH₂CH₂CH₂CH₂)₂₀OH

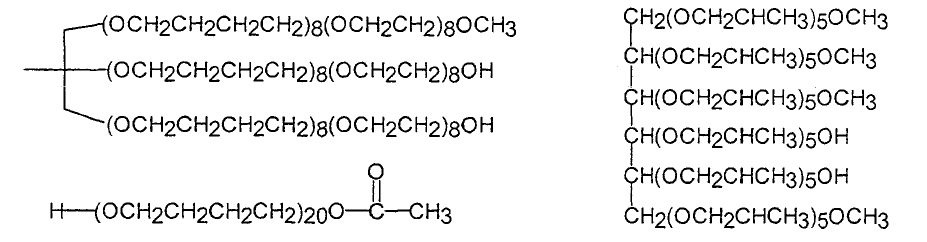

Die erfindungsgemäßen Acryl- und/oder Methacryloylpolyoxyalkylene zeichnen sich dadurch aus, daß wenigstens ein Acryl- und/oder Methacrylsäurerest pro Molekül enthalten ist. Besonders bevorzugt ist es, wenn 5 bis 100 % der Hydroxygruppen acryliert und/oder methacryliert sind.

Die Reihenfolge der Bestandteile der Ausgangskomponenten zur Herstellung der Polyoxyalkylene, wie sie durch die Indices b und d angegeben ist, ist im Sinne der vorliegenden Erfindung beliebig und umfaßt insbesondere Blockcopolymere ebenso wie statistische Polymergruppen sowie deren Kombinationen.

Eine weitere Ausführungsform der Erfindung besteht in dem Verfahren zur Herstellung der obengenannten Reaktionsprodukte.

Das Verfahren zur Herstellung von Acryl- und/oder Methacryloylverbindungen durch Umsetzung von Acryl- und/oder Methacrylsäure und/oder Acryl- und/oder Methacrylsäureestern in Anwesenheit eines die Ver- oder Umesterung katalysierenden Enzyms mit einem Polyoxyalkylen der allgemeinen Formel I, insbesondere bei niedrigen Temperaturen (20 bis 100 °C, bevorzugt 40 bis 70 °C) und milden Bedingungen, ist vorteilhaft aufgrund der helleren Farbe des Produkts, der Vermeidung der Bildung von Nebenprodukten, die andernfalls zum Beispiel von chemischen Katalysatoren stammen können, der unkomplizierten Entfernung des Enzymkatalysators vom Produkt und der Vermeidung unerwünschter und unkontrollierter radikalischer Polymerisation der Acryl- und/oder Methacryloylverbindungen.

Der Kern der vorliegenden Erfindung besteht somit in der Synthese von Acryl- und/oder Methacryloylverbindungen mit Enzymen, insbesondere Hydrolasen, die als Katalysatoren für Ver- beziehungsweise Umesterungreaktionen unter geeigneten Bedingungen fungieren, insbesondere Lipasen, Proteasen und Esterasen.

Durch die Verwendung von Enzymen als Veresterungs- beziehungsweise Umesterungskatalysatoren zur Herstellung von Acryl-und/oder Methacryloylpolyoxyalkylenen können eine Vielzahl der Nachteile der oben genannten und vergleichbarer Verfahren beseitigt werden. Man arbeitet bei niedrigen Temperaturen; somit ist die Gefahr einer unerwünschten Polymerisation der Acryl-und/oder Methacryloylverbindungen stark zurückgedrängt. Es ist kein Einsatz besonders reaktiver Acryl- und/oder Methacrylsäurederivate wie -halogenide oder -anhydride notwendig und das als Katalysator verwendetete Enzym ist leicht abtrennbar.

Die Acrylierung und/oder Methacrylierung verläuft am besten mit hohen Ausbeuten mit Estern der Acryl- und/oder Methacrylsäure als Donormolekülen, insbesondere die Methyl-, Ethyl-, Propyl- oder Butylester.

Enzyme, die beispielsweise als Katalysatoren eingesetzt werden können, sind Hydrolasen, insbesondere Esterasen, Lipasen und Proteasen. Die Enzyme können in reiner Form oder in immobilisierter Form auf einem Träger, auf dem sie chemisch oder physikalisch gebunden sind, eingesetzt werden. Die Menge des Enzymkatalysators beträgt insbesondere, bezogen auf das eingesetzte modifizierte Siloxan, 0,1 bis 20 Gew.-%, vorzugsweise 1 bis 10 Gew.-%. Die Reaktionszeit hängt von der verwendeten Menge und der Aktivität des Enzymkatalysators ab und beträgt beispielsweise bis zu 48 Stunden, vorzugsweise bis zu 24 Stunden.

Um unter einfachen Reaktionsbedingungen schnell zu hohen Umsetzungsgraden zu kommen, ist es vorteilhaft, einen Überschuß von wenigstens 10 Gew.-% Acrylsäure bzw. Methacrylsäure und/oder deren entsprechenden Estern (als Donoren) in der Reaktionsmischung zu verwenden.

Das Produktionssystem läßt sich entweder durch einen Rührkesselreaktor oder einen Festbettreaktor charakterisieren. Der Rührkesselreaktor kann mit einer Vorrichtung zum Abdestillieren des aus dem Acryl- und/oder Methacrylsäuredonor freigesetzten Alkanols beziehungsweise des aus der Acrylsäure und/oder Methacrylsäure freigesetzten Wassers ausgestattet sein.

Die Reaktion wird durchgeführt, bis der gewünschte Umsatz erreicht wird. Eine Reaktionsführung mit gleichzeitiger Destillation wird bevorzugt, weil die Entfernung des Reaktionswassers bzw. Reaktionsalkanols zu höheren Umsätzen in kürzeren Reaktionszeiten aufgrund der Verschiebung des Reaktionsgleichgewichts führt.

Um möglichst hohe Umsetzungsgrade zu erreichen, ist die Entfernung des Reaktionswassers bzw. -alkanols notwendig.

Nach beendeter Umsetzung kann der Enzymkatalysator durch geeignete Maßnahmen, wie Filtrieren oder Dekantieren, abgetrennt und gegebenenfalls mehrmals eingesetzt werden.

Der Festbettreaktor ist mit immobilisierten Enzymen bestückt, wobei die Reaktionsmischung durch die mit Katalysator gefüllte Säule gepumpt wird. Mit einem auf einem Träger immobilisierten Enzym ist es auch möglich, die Reaktion in einem Wirbelbett durchzuführen.

Die Reaktionsmischung kann kontinuierlich durch die Säule gepumpt werden, wobei mit der Fließgeschwindigkeit die Verweilzeit und damit der gewünschte Umsatz zu steuern ist. Es ist auch möglich, die Reaktionsmischung im Kreislauf durch die Säule zu pumpen, wobei auch unter Vakuum das Reaktionswasser bzw. -alkanol gleichzeitig abdestilliert werden kann.

Andere Methoden zur Entfernung des Reaktionswassers bzw. -alkanols können auch verwendet werden, z. B. Absorption oder Pervaporation.

Eine weitere Ausführungsform der vorliegenden Erfindung besteht in der Verwendung der erfindungsgemäßen Acryloyl-und/oder Methacryloylverbindungen als Haupt- oder Nebenbestandteil zur Herstellung und/oder Stabilisierung von Dispersionen (fest/flüssig ebenso wie flüssig/flüssig), als Haupt- oder Nebenbestandteil in strahlenhärtenden Beschichtungen, insbesondere in transparenten Klarlacken und als Haupt- oder Nebenbestandteil zur Herstellung von Polymeren mittels radikalischer Polymerisation.

### Ausführungsbeispiel

282 g eines Polyoxyalkylens der allgemeinen Formel H₂C=CH-CH₂-(OC₂H₄)_{13,5}-OH wurden mit 226 g Butylmethacrylat und 10 g Novozym® 435 auf 70 °C erwärmt. Unter Vakuum (20 bis 40 mbar) wurde das freigesetzte Butanol abdestilliert. Nach 16 h Reaktionszeit betrug der Umsatz 99 %. Der Katalysator wurde abfiltriert und überschüssiges Butylmethacrylat abdestillert. Das Produkt war das reine Methacryloylpolyoxyalkylen.

## Patentansprüche

1. Verfahren zur Herstellung von Acryloyl- und/oder Methacryloylverbindungen von Polyoxyalkylenen durch Ver- oder Umesterung von Acryl- und/oder Methacrylsäure oder Acryl- und/oder Methacrylsäureestern mit Polyoxyalkylenen der allgemeinen Formel (I) 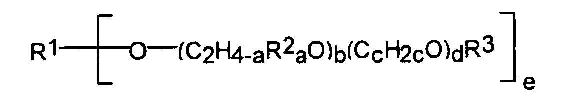 wobei
R¹ = ein Wasserstoff, ein e-bindiger, gegebenenfalls verzweigter, cyclischer, ungesättigter und/oder aromatischer Kohlenwasserstoffrest, ein gegebenenfalls substituierter Aromat, ein Kohlenhydrat oder Kohlenhydratderivat ist,
R² = gleiche oder verschiedene Alkylreste oder Alkylenreste mit 1 bis 24 Kohlenstoffatomen oder gegebenenfalls substituierte Phenylreste mit bis zu 24 Kohlenstoffatomen,
R³ = ein Wasserstoffrest oder ein einwertiger organischer Rest,
a = 0 bis 3,
b = 0 bis 100,
c = 2 bis 12,
d = 0 bis 100,
e = 1 bis 30,
die Summe (b + d) = 4 bis 200 ist,
wobei pro Molekül wenigstens eine OH-Gruppe vorhanden ist,
und die Reihenfolge der Polyoxyalkylensegmente (C₂H₄₋ₐR²ₐO)_{b} und (C_{c}H_{2c}O)_{d} beliebig ist, **dadurch gekennzeichnet, dass** man die Ver- oder Umesterung in Anwesenheit eines die Ver- oder Umesterung katalysierenden Enzyms durchführt.

2. Verfahren gemäß Anspruch 1, **dadurch gekennzeichnet, daß** die Summe (b + d) = 8 bis 120 ist.

3. Verfahren nach Anspruch 1 oder 2, **dadurch gekennzeichnet, daß** man als Enzym Hydrolasen, insbesondere immobilisierte Enzyme einsetzt, die ausgewählt sind aus Lipasen, Esterasen oder Proteasen.

4. Verfahren nach Anspruch 1 bis 3, **dadurch gekennzeichnet, daß** man die Reaktionen bei einer Temperatur im Bereich von 20 bis 100 °C, insbesondere 40 bis 70 °C durchführt.

5. Verfahren nach Anspruch 1 bis 4, **dadurch gekennzeichnet, daß** man als Acryl- und/oder Methacrylsäuredonor Methyl-, Ethyl-, Propyl- oder Butylester der Methacryl- und/oder Acrylsäure einsetzt.

6. Verwendung von Acryloyl- und/oder Methacryloylverbindungen gemäß den Ansprüchen 1 bis 5 als Haupt- oder Nebenbestandteil zur Herstellung und/oder Stabilisierung von Dispersionen.

## Claims

1. Process for preparing acryloyl and/or methacryloyl compounds of polyoxyalkylenes by esterifying or transesterifying acrylic acid and/or methacrylic acid or acrylic esters and/or methacrylic esters with polyoxyalkylenes of the general formula (I) 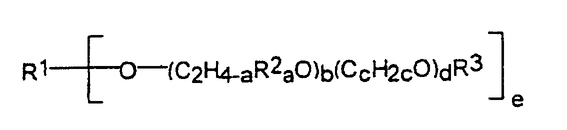 where
R¹ = hydrogen, an e-valent, optionally branched, cyclic, unsaturated and/or aromatic hydrocarbon radical, an optionally substituted aromatic, a carbohydrate or carbohydrate derivative,
R² = identical or different alkyl radicals or alkylene radicals having 1 to 24 carbon atoms or optionally substituted phenyl radicals having up to 24 carbon atoms,
R³ = a hydrogen radical or a monovalent organic radical,
a = 0 to 3,
b = 0 to 100,
c = 2 to 12,
d = 0 to 100,
e = 1 to 30,
the sum of (b + d) = 4 to 200,
at least one OH group being present per molecule, and the sequence of the polyoxyalkylene segments (C₂H₄₋ₐR²ₐO)_{b} and (C_{c}H_{2c}O)_{d} is as desired, **characterized in that** the esterification or transesterification is carried out in the presence of an enzyme which catalyses the esterification or transesterification.

2. Process according to Claim 1, **characterized in that** the sum of (b + d) = 8 to 120.

3. Process according to Claim 1 or 2, **characterized in that** the enzymes used are hydrolases, especially immobilized enzymes which are selected from lipases, esterases or proteases.

4. Process according to Claim 1 to 3, **characterized in that** the reactions are carried out at a temperature in the range of 20 to 100°C, in particular 40 to 70°C.

5. Process according to Claim 1 to 4, **characterized in that** the acrylic acid and/or methacrylic acid donor used is the methyl, ethyl, propyl or butyl ester of methacrylic acid and/or of acrylic acid.

6. Use of acryloyl and/or methacryloyl compounds according to Claims 1 to 5 as a main or secondary constituent for preparing and/or stabilizing dispersions.

## Revendications

1. Procédé de préparation de composés d'acryloyle et/ou de méthacryloyle de polyoxyalkylènes par estérification ou transestérification d'acide acrylique et/ou méthacrylique ou d'esters d'acide acrylique et/ou méthacrylique avec des polyoxyalkylènes de la formule générale (I) 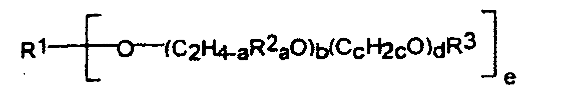 dans laquelle
R¹ = un atome d'hydrogène, un radical hydrocarboné à liaison E, éventuellement ramifié, cyclique, insaturé et/ou aromatique, un aromatique éventuellement substitué, un hydrate de carbone ou un dérivé d'hydrate de carbone,
R² = des radicaux alkyle ou alkylène identiques ou différents, comportant de 1 à 24 atomes de carbone, ou des radicaux phényle éventuellement substitués comportant de 1 à 24 atomes de carbone,
R³ = un atome d'hydrogène ou un radical organique monovalent,
a = 0 à 3,
b = 0 à 100,
c = 2 à 12,
d = 0 à 100,
e = 1 à 30,
la somme (b + d) valant 4 à 200,
au moins un groupe OH étant présent par molécule,
et la séquence des segments de polyoxyalkylène (C₂H₄₋ₐR²ₐO)_{b} et (C_{c}H_{2c}O)_{d} étant quelconque, **caractérisé en ce que** l'on entreprend l'estérification ou la transestérification en présence d'une enzyme catalysant l'estérification ou la transestérification.

2. Procédé selon la revendication 1, **caractérisé en ce que** la somme (b + d) a une valeur de 8 à 120.

3. Procédé selon la revendication 1 ou 2, **caractérisé en ce que** l'on met en oeuvre en tant qu'enzymes des hydrolases, en particulier des enzymes immobilisées, qui sont choisies parmi des lipases, des estérases ou des protéases.

4. Procédé selon les revendications 1 à 3, **caractérisé en ce que** l'on entreprend les réactions à une température de l'ordre de 20 à 100°C, en particulier de 40 à 70°C.

5. Procédé selon les revendications 1 à 4, **caractérisé en ce que** l'on met en oeuvre en tant que donneurs d'acide acrylique et/ou méthacrylique un ester méthylique, éthylique, propylique ou butylique de l'acide acrylique et/ou méthacrylique.

6. Utilisation de composés d'acryloyle et/ou de méthacryloyle selon les revendications 1 à 5 en tant que constituants principaux ou secondaires pour la préparation et/ou la stabilisation de dispersions.
